# EUROPÄISCHE PATENTSCHRIFT

(11) **EP 0 388 589 B1**
(45) Veröffentlichungstag und Bekanntmachung des Hinweises auf die Patenterteilung: **21.07.1993**
(21) Anmeldenummer: 90101268.2
(22) Anmeldetag: 23.01.1990
(51) Int. Cl.: G01N 33/18

(54) **Verfahren zum Bestimmen von zersetzungsfähigen Kohlenstoffverbindungen in Wasser**
Method for determining degradable organic matter in water
Procédé pour déterminer des composés organiques dégradables dans l'eau

(30) Priorität: 21.03.1989 DE 3909240
(43) Veröffentlichungstag der Anmeldung: 26.09.1990
(73) Patentinhaber: HÜLS AKTIENGESELLSCHAFT, 45764 Marl (DE)
(72) Erfinder: Duve, Hans, Dipl.-Ing., D-4408 Dülmen (DE)

(56) Entgegenhaltungen:
- EP-A- 0 057 390
- DE-A- 3 015 663
- FR-A- 2 408 139
- ISA TRANSACTIONS, Band 7, Nr. 4, 1968, Seiten 267-272; R.H. JONES et al.: "Application of a high-sensitivity total-organic-carbon analyzer"

## Beschreibung

Die Erfindung betrifft ein Verfahren zum kontinuierlichen und quantitativen Untersuchen von Wasser, das zersetzungsfähige Kohlenstoffverbindungen enthält. Es verfolgt den Zweck, den gesamten Gehalt an organischen und anorganischen Kohlenstoffverbindungen quantitativ zu bestimmen, auch wenn die organischen Kohlenstoffverbindungen teilweise oder ganz flüchtig sind.

Bei den bekannten Nachweisverfahren, z. B. nach DIN 38 409, Teil 3, wird der anorganisch gebundene Kohlenstoff (TIC - Total Inorganic Carbon) in Form von Kohlendioxid (CO₂) aus der angesäuerten Wasserprobe mit Hilfe eines Gasstromes ausgetrieben. Die in der ausgegasten Wasserprobe enthaltenen organischen Verbindungen werden nach einem geeigneten Verfahren zu CO₂ oxidiert, und die Menge an dabei entstandenem CO₂ wird nachgewiesen. Enthält die zu untersuchende Wasserprobe leichtflüchtige organische Verbindungen, müssen diese Stoffe entweder gesondert bestimmt werden, oder man wendet eine Differenzmethode an. Bei der Differenzmethode wird zuerst der Gehalt an Gesamtkohlenstoff (TC - Total Carbon) bestimmt und anschließend der Gehalt an anorganisch gebundenem Kohlenstoff (TIC) abgezogen. Diese Differenz ist der Gehalt an organisch gebundenem Kohlenstoff (TOC - Total Organic Carbon). Diese Arbeitsweise ist bei der Bestimmung von Einzelproben möglich. Für Meßverfahren, bei denen der Kohlenstoff aller ausgasbaren organischen Verbindungen (POC - Purgible Organic Carbon) miterfaßt wird, werden die Wasserprobe und das Strippgas hinter dem Ausgasegefäß mit unterschiedlichen Methoden separat untersucht.

Damit stellt sich die Aufgabe, ein Verfahren zu finden, mit dem man einen wäßrigen Probestrom kontinuierlich auf den Gehalt an organisch und anorganisch gebundenem Kohlenstoff untersuchen kann, unabhängig von der Flüchtigkeit der im Probestrom enthaltenen organischen Verbindungen.

Die Aufgabe wird erfindungsgemäß gelöst durch ein Verfahren mit den in Anspruch 1 angegebenen kennzeichnenden Merkmalen.

Aus dem wäßrigen Probestrom werden in an sich bekannter Weise das durch Ansäuern aus anorganischen Kohlenstoffverbindungen entstandene CO₂ und die enthaltenen flüchtigen organischen Verbindungen mit einem Transportgas kontinuierlich in diesen Gasstrom überführt. Das Transportgas muß vor dem Einleiten in das Ausgasegefäß frei sein von kohlenstoffhaltigen Stoffen jeder Art. Dieses Transportgas wird nach dem Ausgasegefäß durch den Zersetzungsreaktor geleitet. Der ausgegaste wäßrige Probestrom wird mit den darin enthaltenen nichtflüchtigen organischen Verbindungen ebenfalls in einen Zersetzungsreaktor eingebracht, der von dem Zersetzungsreaktor für das Transportgas aus dem Ausgasegefäß verschieden sein kann; in diesem Fall wird der Zersetzungsreaktor für den ausgegasten, flüssigen Probestrom mit einem separaten Transportgasstrom beschickt. Das aus dem einen Zersetzungsreaktor oder beiden Zersetzungsreaktoren abgeleitete Transportgas wird jeweils einem CO₂-Detektor zugeführt. Bevorzugt werden das Transportgas und der wäßrige Probestrom aus dem Ausgasegefäß in denselben Zersetzungsreaktor geleitet, und das im Transportgas nach dem Ausgasegefäß enthaltene CO₂, das aus anorganischen Kohlenstoffverbindungen stammt, wird gegebenenfalls mit einem CO₂-Detektor nachgewiesen und mit einem CO₂-Adsorber entfernt. Dann enthält das Transportgas nur die flüchtigen organischen Kohlenstoffverbindungen, die anschließend im Zersetzungsreaktor zersetzt werden. Verzichtet man zwischen Ausgasegefäß und Zersetzungsreaktor auf den CO₂-Adsorber im Transportgasstrom, dann erhält man am CO₂-Detektor hinter dem Zersetzungsreaktor einen CO₂-Gehalt, der dem Gesamtkohlenstoffgehalt (TC) des Probestromes entspricht. Wenn man den CO₂-Gehalt im Transportgas, das das Ausgasegefäß verläßt, durch einen CO₂-Detektor direkt am Ausgang des Ausgasegefäßes nachweist und das Transportgas über einen CO₂-Adsorber leitet, erhält man den TC-Gehalt des Probestromes durch Addition der in den beiden CO₂-Detektoren nachgewiesenen CO₂-Gehalte.

Um die Ansprechzeit des erfindungsgemäßen Meßverfahrens bei der Untersuchung eines stark organisch belasteten Probestromes nicht durch Verringerung des Probestromes zu vergrößern, kann der Probestrom hinter dem Ausgasegefäß aufgeteilt werden; die Verringerung des Probestromes, der durch das Ausgasegefäß in den Zersetzungsreaktor geleitet wird, ist zwar möglich, wenn man den Zersetzungsreaktor vor einer Überlastung durch einen stark organisch belasteten Probestrom schützen will, jedoch führt dies in jedem Fall zu einer längeren Ansprechzeit. Bei der Aufteilung des Probestromes hinter dem Ausgasegefäß wird der eine Teil in den Zersetzungsreaktor geleitet, der andere Teil wird vor dem Zersetzungsreaktor verworfen. In diesem Fall wird das Transportgas hinter dem Ausgasegefäß im gleichen prozentualen Verhältnis ausgeteilt. Der verworfene Anteil an Transportgas wird durch Zuführen von Gas vor dem Ausgasegefäß ersetzt. Da die Mengenströme an flüssigem Probegut und an Transportgas, welche in den Zersetzungsreaktor eingeleitet werden, äquivalente Teilmengen - bezogen auf die Verhältnisse im Ausgasegefäß - sind, bleibt der über den Anteil des flüssigen Probestromes und über den Anteil des Transportgases dem Zersetzungsreaktor zugeführten Gehalt an zersetzungsfähigen Kohlenstoffverbindungen unverändert und damit die Analyse des wäßrigen Probestromes weiterhin möglich. An Stelle der Teilung des Probestromes und des Transportgasstromes jeweils hinter dem Ausgasegefäß kann auch das Volumen des Ausgasegefäßes der gewünschten Verweilzeit des Probestromes im Ausgasegefäß angepaßt werden.

Wird der Probestrom hinter dem Ausgasegefäß vollständig verworfen und das Transportgas, welches das Ausgasegefäß verläßt, nur über einen CO₂-Adsorber in den Zersetzungsreaktor geführt, entspricht der CO₂-Gehalt im Transportgas nach dem Zersetzungsreaktor dem Gehalt an flüchtigen organischen Verbindungen (POC) im Probestrom.

Als CO₂-Adsorber sind Natronkalk und Natronasbest geeignet. Werden als CO₂-Adsorber gekörnter Natronkalk oder Natronkalk-Plätzchen eingesetzt, ist es zweckmäßig, diese vorher mit Wasser anzufeuchten. Durch das einmalige Anfeuchten wird die Adsorption von organischen Kohlenstoffverbindungen aus dem Transportgas verhindert. Diese Eigenschaft der Natronkalk-Plätzchen bleibt überraschenderweise nach dem Abtrocknen im Transportgasstrom erhalten.

Die zum Zersetzen der anorganischen Kohlenstoffverbindungen benötigte Säure wird zweckmäßigerweise mittels einer Pumpe in den Strom des Probegutes eingespeist, bevor dieser in das Ausgasegefäß eintritt. Die Bedingungen im Ausgasegefäß müssen so gewählt werden, daß das aus den anorganischen Kohlenstoffverbindungen entstandene CO₂ vollständig in den Transportgasstrom übergeht.

Die Art des Zersetzungsreaktors ist frei wählbar (u.a. photochemisch, thermisch), unter der Bedingung, daß bei der Zersetzung der organischen Kohlenstoffverbindungen der Kohlenstoff nur als Kohlendioxid im Transportgas erscheint.

Als CO₂-Detektoren werden Infrarot-Analysatoren, auf CO₂ ansprechende gassensitive Elektroden oder CO₂-Sensoren verwendet; es können aber prinzipiell alle Analysatoren eingesetzt werden, mit denen man geringe CO₂-Konzentrationen in der Gasphase nachweisen kann. Falls der CO₂-Nachweis z. B. mit einem IR-Analysator durch einen zu hohen Gehalt an Wasserdampf (aus dem Ausgasegefäß oder dem Zersetzungsreaktor) oder an Ozon (ggf. aus dem Zersetzungsreaktor) gestört wird, ist vor die CO₂-Detektoren eine Gastrocknungsvorrichtung und/oder ein Ozon-Zersetzer vorzuschalten.

Als Transportgas wird bevorzugt Luft eingesetzt, die frei von kohlenstoffhaltigen Substanzen ist. Vorteilhaft ist jedoch ein von molekularem Sauerstoff freies Transportgas, vorzugsweise Stickstoff, oder ein Edelgas, wie Helium oder Argon.

Das erfindungsgemäße Verfahren hat folgende Vorteile:
- Die im wäßrigen Probestrom enthaltenen zersetzungsfähigen organischen Kohlenstoffverbindungen werden quantitativ und kontinuierlich nachgewiesen, auch wenn diese teilweise oder ganz aus dem wäßrigen Probestrom im Ausgasegefäß entfernt werden, ohne daß dieser Nachweis durch einen gegebenenfalls auch großen Gehalt an anorganischen Kohlenstoffverbindungen im Probestrom gestört wird.
- Wird nur das Transportgas mit den darin enthaltenen ausgasbaren organischen Verbindungen in den Zersetzungsreaktor geleitet, kann der POC-Wert des Probestromes kontinuierlich bestimmt werden.
- Der im Probestrom enthaltene anorganische Kohlenstoff (TIC) kann kontinuierlich und parallel mitbestimmt werden.
- Über die Größe der drei Stoffströme
   - Probestrom in das Ausgasegefäß
   - Probestrom in den Zersetzungsreaktor
   - Transportgasstrom in den Zersetzungsreaktor
   kann die Empfindlichkeit des Meßverfahrens an den Kohlenstoffgehalt des Probestromes angeglichen werden.
- Die benutzte Vorrichtung ist eichfähig entsprechend DIN 38 409, Teil 3, Abschnitt 12, wenn man eine Eichlösung als Probestrom verwendet, für die Konzentration und Art der enthaltenen Verbindung bekannt sind.
- Eine Konzentration an C-Verbindungen bis hin zum ppb-Bereich läßt sich einwandfrei nachweisen.

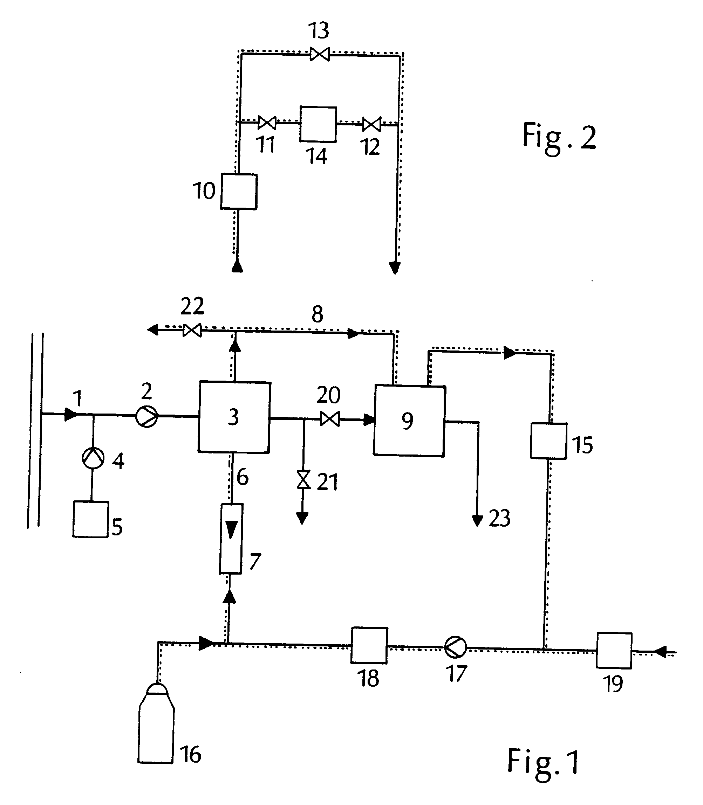

Das erfindungsgemäße Verfahren kann beispielsweise mittels der in Figur 1 dargestellten Vorrichtung durchgeführt werden.

Über eine Zuleitung (1) wird der Probestrom aus dem zu untersuchenden Wasser abgezweigt und mit einer Dosierpumpe (2) kontinuierlich in das Ausgasegefäß (3) gefördert. In die Zuleitung (1) wird mit einer Dosierpumpe (4) Säure aus einem Vorratsgefäß (5) zugegeben, wodurch der pH-Wert im Ausgasegefäß auf den gewünschten Wert eingestellt wird. Durch das angesäuerte Wasser im Ausgasegefäß wird über Leitung (6) und Durchflußmessung (7) ein definierter Transportgasstrom hindurchgeleitet und nach dem Verlassen des Ausgasegefäßes über die Leitung (8) direkt durch einen Zersetzungsreaktor (9) geführt. Die Zuführung des Transportgases vom Ausgasegefäß zum Zersetzungsreaktor kann wahlweise (siehe Figur 2) nur über CO₂-Detektor (10) (Ventile (11) und (12) zu, Ventil (13) auf) erfolgen, oder unter Zwischenschaltung eines CO₂-Adsorbers (14) (Ventile (11) und (12) auf, Ventil (13) zu). Das den Zersetzungsreaktor verlassende Transportgas wird einem CO₂-Detektor (15) zugeführt. Das Transportgas für die Zuführung in das Ausgasegefäß kann wahlweise Kohlenstoff-freies Frischgas (16) sein, oder hinter dem CO₂-Detektor (15) mit einer Pumpe (17) über einen CO₂-Adsorber (18) rückgeführtes Transportgas, oder Luft, die über den CO₂-Adsorber (19) in den Transportgasstrom eingezogen wird. Der angesäuerte und ausgegaste Probestrom kann hinter dem Ausgasegefäß wahlweise in den Zersetzungsreaktor geleitet werden (Ventil (20) auf, Ventil (21) zu) oder vor dem Zersetzungsreaktor verworfen werden (Ventil (20) zu, Ventil (21) auf). Über teilgeöffnete Ventile (20) und (21) kann der Probestrom beliebig aufgeteilt werden. Das Ventil (22) dient dazu, das Transportgas entsprechend der Aufteilung des Probestromes - mittels der Ventile (20) und (21) - ebenfalls aufzuteilen. Zum Messen der ausgasbaren organischen Kohlenstoffverbindungen allein werden die Ventile (20) und (22) geschlossen, während Ventil (21) voll geöffnet ist.

Bei der Verwendung eines thermischen Zersetzungsreaktors wird der zugeführte Probestrom vollständig verdampft, wobei sich die kohlenstoffhaltigen Verbindungen in CO₂ umwandeln. Wird ein photolytischer Zersetzungsreaktor eingesetzt, wird der Wasserstand in diesem Reaktor über den Überlauf (23) konstant gehalten.

Das erfindungsgemäße Verfahren wird durch folgende Beispiele erläutert.

### Beispiel 1

Bestimmung des TOC-Gehaltes eines Wassers mit bekanntem Kohlenstoffgehalt.

Aus einem Vorratsgefäß wird der zu untersuchende Feststoff-freie Wasserstrom entnommen und mit einem Durchsatz von 500 ml/Stunde in das Ausgasegefäß (Inhalt ca. 50 ml) gepumpt. Das Probegut enthält 0,54 mg Benzol/Liter und 1,06 mg Kaliumhydrogenphthalat/Liter als organische Kohlenstoffverbindungen sowie 70 mg Natriumhydrogencarbonat/Liter als anorganische Kohlenstoffverbindung. In diesem Wasser sind also 1,0 mg organischer Kohlenstoff und 10 mg anorganischer Kohlenstoff pro Liter enthalten.

Vor der Dosierpumpe wird der Probestrom durch Zugabe von 3%iger Phosphorsäure auf einem pH-Wert von 2,5 bis 3,5 gehalten und nach dem Ausgasegefäß durch den Zersetzungsreaktor geleitet. Das Transportgas (Luft) wird mit einem Durchsatz von 100 Liter/Stunde durch das Ausgasegefäß geleitet, über den CO₂-Adsorber geführt und am Boden des photolytischen Zersetzungsreaktors in die darin befindliche flüssige Phase eingeperlt. Als CO₂-Adsorptionsmittel werden Natronkalk-Plätzchen mit Indikator (Art. 6839, Merck Darmstadt) verwendet. Die Natronkalk-Plätzchen wurden vor dem Einfüllen in das CO₂-Adsorptionsgefäß mit Wasser gesättigt, und die überschüssige Flüssigkeit wurde abdekantiert. Der Zersetzungsreaktor (Inhalt ca. 0,4 Liter) ist mit einer Niederdruck-Hg-Lampe mit einer Leistung von 160 Watt bestückt. Der Wasserstand im UV-Reaktor wird über einen Ablauf konstant gehalten. Das Transportgas verläßt den UV-Reaktor im oberen Teil, wird über einen Kühler getrocknet, in einem Ofen von Ozon befreit, und durchläuft dann kontinuierlich einen IR-Analysator. Der aus dem IR-Analysator abfließende Transportgasstrom wird über einen weiteren CO₂-Adsorber in das Ausgasegefäß zurückgeführt.

Bei der Untersuchung des obengenannten Probestromes wird eine CO₂-Konzentration im Transportgas nach dem Zersetzungsreaktor von 9 Vol.-ppm gemessen. Dieses entspricht einem TOC-Gehalt des Probestromes von 0,96 mg/Liter und damit einer Wiederfindungsrate von 96 % des organisch gebundenen Kohlenstoffs, obwohl in dem vorgelegten Probestrom neben dem organisch gebundenen Kohlenstoff eine zehnmal größere Menge an anorganisch gebundenem Kohlenstoff vorliegt und der organisch gebundene Kohlenstoff zur Hälfte in einer flüchtigen Verbindung anwesend ist.

### Beispiel 2

Bestimmung des TC-Gehaltes eines Wassers mit bekanntem Kohlenstoffgehalt.

Die Untersuchung nach Beispiel 1 wird wiederholt, jedoch wird das Transportgas hinter dem Ausgasegefäß nicht über den CO₂-Adsorber geleitet, sondern direkt in den Zersetzungsreaktor geführt. Die anderen Parameter entsprechen denen im Beispiel 1.

Es wird eine CO₂-Konzentration von 98 Vol.-ppm gemessen. Dieses entspricht einem TC-Gehalt des Probestromes von 10,5 mg/Liter und damit einer Wiederfindungsrate von 95 % des gesamten gebundenen Kohlenstoffs.

### Beispiel 3

Bestimmung des POC-Gehaltes eines Wassers mit bekanntem Kohlenstoffgehalt.

Die Untersuchung nach Beispiel 1 wird wiederholt, jedoch wird der Probestrom hinter dem Ausgasegefäß vollständig verworfen. Der Zersetzungsreaktor ist mit Wasser gefüllt, das den Zersetzungsreaktor nicht mehr durchströmt. Die anderen Parameter entsprechen denen in Beispiel 1.

Es wird eine CO₂-Konzentration im Transportgas nach dem Zersetzungsreaktor von 4,3 Vol.-ppm gemessen. Dieses entspricht einem ausgasbaren organischen Kohlenstoffgehalt (POC) des Probestromes von 0,46 mg/Liter und damit einer Wiederfindungsrate von 92 % des Kohlenstoffs in der flüchtigen Kohlenstoffverbindung.

### Beispiel 4

Bestimmung des TIC- und TOC-Gehaltes eines Wassers mit unbekanntem Kohlenstoffgehalt.

Aus einer Hauptleitung wird der zu untersuchende Feststoff-freie Probestrom abgezweigt und mit einem Durchsatz von 500 ml/Stunde in das Ausgasegefäß (Inhalt ca. 50 ml) gepumpt. Vor der Dosierpumpe wird der Probestrom durch Zugeben von 3%iger Phosphorsäure auf einem pH-Wert von 2,7 bis 3,2 gehalten. Der gesamte Probestrom wird nach dem Ausgasegefäß in den photolytischen Zersetzungsreaktor geleitet. Das Transportgas (Luft) wird mit einem Durchsatz von 100 Liter/Stunde durch das Ausgasegefäß geleitet, über einen Kühler getrocknet und durchläuft dann einen IR-Analysator als CO₂-Detektor. Der Transportgasstrom wird weiter über einen CO₂-Adsorber geführt und am Boden des photolytischen Zersetzungsreaktors in die darin befindliche flüssige Phase eingeperlt. Anschließend wird das Transportgas mit einem Kühler getrocknet und in einem Ofen von Ozon befreit; es durchläuft dann kontinuierlich einen anderen IR-Analysator. Das aus diesem IR-Analysator abgeführte Transportgas wird über einen weiteren CO₂-Adsorber in das Ausgasegefäß zurückgeführt. Die weiteren Bedingungen entsprechen denen im Beispiel 1.

An dem IR-Analysator hinter dem Ausgasegefäß wird ein CO₂-Gehalt von 24 Vol.-ppm gemessen. Dieses entspricht einem TIC-Gehalt des Probegutes von 2,6 mg/Liter. Am IR-Analysator hinter dem Zersetzungsreaktor wird ein CO₂-Gehalt von 37 Vol.-ppm gemessen. Dieses entspricht einem TOC-Gehalt des Probegutes von 4,0 mg/Liter, welcher unabhängig von der tatsächlich erreichten Ausgasung der unbekannten und gegebenenfalls flüchtigen organischen Kohlenstoffverbindungen ist.

## Patentansprüche

1. Verfahren zum kontinuierlichen und quantitativen Bestimmen von organischen und anorganischen Kohlenstoffverbindungen in Wasser, unter vollständiger Miterfassung aller ausgasbaren Kohlenstoffverbindungen, gekennzeichnet durch,
- kontinuierliches Durchleiten einer Probe des zu untersuchenden Wassers durch ein Ausgasegefäß,
- Einstellen des pH-Wertes des zu untersuchenden Wassers im Ausgasegefäß auf einen Wert im Bereich 2 bis 4,
- kontinuierliches Durchleiten eines Transportgases durch das Ausgasegefäß, wobei das Transportgas vor dem Einleiten frei von kohlenstoffhaltigen Substanzen ist,
- kontinuierliches Durchleiten dieses Transportgases nach dem Ausgasegefäß durch einen Zersetzungsreaktor,
- kontinuierliches Durchleiten des Transportgases nach dem Zersetzungsreaktor durch einen Kohlendioxid-Detektor.

2. Verfahren nach Anspruch 1, gekennzeichnet durch
- kontinuierliches Durchleiten des zu untersuchenden Wassers oder Teilmengen davon nach dem Ausgasegefäß durch den Zersetzungsreaktor, in den auch das Transportgas aus dem Ausgasegefäß eingeleitet wird.

3. Verfahren nach den Ansprüchen 1 und 2, gekennzeichnet durch
- kontinuierliches Durchleiten des Transportgases oder Teilmengen davon nach dem Ausgasegefäß durch einen Kohlendioxid-Adsorber vor dem Durchleiten dieses Gases durch den Zersetzungsreaktor, gegebenenfalls nach vorherigem Durchleiten durch einen Kohlendioxid-Detektor.

4. Verfahren nach den Ansprüchen 1 bis 3, gekennzeichnet durch
- Teilen des wäßrigen Probestromes hinter dem Ausgasegefäß in zwei Teilströme, von denen der eine Teilstrom in den Zersetzungsreaktor geführt wird und der andere Teilstrom verworfen wird,
- Teilen des Transportgases hinter dem Ausgasegefäß in zwei Teil ströme, von denen der eine Teilstrom in den Zersetzungsreaktor geleitet wird und der andere Teilstrom verworfen wird,
- wobei der in den Zersetzungsreaktor geleitete wäßrige Probeteilstrom bzw. Transportgasteilstrom jeweils derselbe Anteil des in das Ausgasegefäß eingeleiteten wäßrigen Probestromes bzw. Transportgasstromes ist.

5. Verfahren nach den Ansprüchen 1 bis 3, gekennzeichnet durch
- vollständiges Verwerfen des wäßrigen Probestromes hinter dem Ausgasegefäß.

6. Verfahren nach den Ansprüchen 1 bis 5, gekennzeichnet durch
- Messen des Kohlendioxid-Gehaltes in dem Transportgas, das das Ausgasegefäß verläßt.

7. Verfahren nach den Ansprüchen 1 bis 6, gekennzeichnet durch
- kontinuierliches Rückführen des Transportgases aus dem Zersetzungsreaktor über den Kohlendioxid-Detektor und über einen Kohlendioxid-Adsorber in das Ausgasegefäß.

8. Verfahren nach den Ansprüchen 1 bis 7, gekennzeichnet durch
- Verwenden von befeuchtetem Natronkalk als Kohlendioxid-Adsorber.

9. Verfahren nach den Ansprüchen 1 bis 8, gekennzeichnet durch
- Verwenden von Luft als Transportgas, oder
- Verwenden eines von molekularem Sauerstoff freien Transportgases, vorzugsweise Stickstoff oder ein Edelgas.

## Claims

1. A process for the continuous and quantitative determination of organic and inorganic carbon compounds in water, with a complete inclusion in the determination of all purgeable carbon compounds, characterised by
- continuously passing a sample of the water to be analysed through a purging vessel,
- adjusting the pH of the water to be analysed in the purging vessel to a value in the range from 2 to 4,
- continuously passing a transport gas through the purging vessel, the transport gas prior to the introduction being free from carbon-containing substances,
- continuously passing this transport gas, after the purging vessel, through a decomposition reactor,
- continuously passing the transport gas, after the decomposition reactor, through a carbon dioxide detector.

2. A process according to claim 1, characterised by
- continuously passing the water to be analysed or part-quantities thereof, after the purging vessel, through the decomposition reactor into which the transport gas from the purging vessel is also introduced.

3. A process according to either of claims 1 and 2, characterised by
- continuously passing the transport gas or part-quantities thereof, after the purging vessel, through a carbon dioxide adsorber prior to passing this gas through the decomposition reactor, if desired after previously passing the gas through a carbon dioxide detector.

4. A process according to any of claims 1 to 3, characterised by
- dividing the aqueous sample stream downstream of the purging vessel into two part-streams, of which the one part-stream is directed into the decomposition reactor and the other part-stream is discarded,
- dividing the transport gas downstream of the purging vessel into two part-streams, of which the one part-stream is conducted into the decomposition reactor and the other part-stream is discarded,
- the aqueous sample part-stream or transport gas part-stream conducted into the decomposition reactor being in each case the sample fraction of the aqueous sample stream or transport gas stream, respectively, introduced into the purging vessel.

5. A process according to any of claims 1 to 3, characterised by
- completely discarding the aqueous sample stream downstream of the purging vessel.

6. A process according to any of claims 1 to 5, characterised by
- measuring the carbon dioxide content of the transport gas which leaves the purging vessel.

7. A process according to any of claims 1 to 6, characterised by
- continuously returning the transport gas from the decomposition reactor into the purging vessel via the carbon dioxide detector and via a carbon dioxide adsorber.

8. A process according to any of claims 1 to 7, characterised by
- using moist soda lime as carbon dioxide adsorber.

9. A process according to any of claims 1 to 8, characterised by
- using air as transport gas, or
- using a transport gas free from molecular oxygen, preferably nitrogen or a noble gas.

## Revendications

1. Procédé pour la détermination continue et quantitative de composés organiques et minéraux carbonés dans de l'eau avec saisie conjointe complète de tous les composés carbonés dégazables, caractérisé par le fait :
- que l'on fait passer en continu un échantillon de l'eau à examiner à travers un récipient de dégazage,
- que l'on ajuste la valeur du pH de l'eau à examiner dans le récipient de dégazage à une valeur comprise dans un domaine de 2 à 4,
- que l'on fait passer en continu un gaz d'entraînement à travers le récipient de dégazage, le gaz d'entraînement étant avant son introduction exempt de substances renfermant du carbone,
- que l'on fait passer en continu ce gaz d'entraînement, après le récipient de dégazage, à travers un réacteur de décomposition,
- que l'on fait passer en continu le gaz d'entraînement, après le réacteur de décomposition, à travers un détecteur de dioxyde de carbone.

2. Procédé selon la revendication 1, caractérisé par le fait :
- que l'on fait passer en continu de l'eau à examiner ou de quantités partielles de celle-ci, après le récipient de dégazage, à travers le réacteur de décomposition dans lequel on introduit également le gaz d'entraînement provenant du récipient de dégazage.

3. Procédé selon les revendications 1 et 2, caractérisé par le fait :
- que l'on fait passer en continu du gaz d'entraînement ou des quantités partielles de ce dernier, après le récipient de dégazage, à travers un adsorbeur de dioxyde de carbone, ce avant le passage de ce gaz travers le réacteur de décomposition, le cas échéant après passage préalable à travers un détecteur de dioxyde de carbone.

4. Procédé selon les revendications 1 à 3, caractérisé par le fait :
- que l'on subdivise le courant d'échantillon aqueux, derrière le récipient de dégazage, en deux courants partiels dont l'un est envoyé dans le réacteur de décomposition et l'autre est jeté,
- que l'on subdivise les gaz d'entraînement, derrière le récipient de dégazage, en deux courants partiels dont l'un est envoyé dans le réacteur de décomposition et l'autre est jeté,
- tandis que le courant partiel d'échantillon aqueux passant dans le réacteur de décomposition et le courant partiel de gaz d'entraînement sont chacun la même fraction du courant d'échantillon aqueux et du courant de gaz d'entraînement introduit dans le récipient de dégazage.

5. Procédé selon les revendications 1 à 3, caractérisé par le fait :
-que l'on jette en totalité le courant d'échantillon aqueux derrière le récipient de dégazage.

6. Procédé selon les revendications 1 à 5, caractérisé par le fait :
- que l'on mesure la teneur en dioxyde de carbone dans le gaz transporteur qui quitte le récipient de dégazage.

7. Procédé selon les revendications 1 à 6, caractérisé par le fait :
- que l'on renvoie en continu, dans le récipient de dégazage, le gaz d'entraînement provenant du réacteur de décomposition en passant par le détecteur de dioxyde de carbone et par un adsorbeur de dioxyde de carbone.

8. Procédé selon les revendications 1 à 7, caractérisé par le fait :
- que l'on utilise en tant qu'adsorbeur de dioxyde de carbone, de la chaux sodée humide.

9. Procédé selon les revendications 1 à 8, caractérisé par le fait :
- que l'on utilise de l'air comme gaz d'entraînement, ou
- que l'on utilise un gaz d'entraînement exempt d'oxygène moléculaire, de préférence l'azote ou un gaz rare.
